(19) Europäisches Patentamt
European Patent Office
Office européen des brevets

(11) Publication number: **0 155 040**
**B1**

(12) # EUROPEAN PATENT SPECIFICATION

(45) Date of publication of patent specification: **27.04.88**

(51) Int. Cl.⁴: **C 07 C 143/20,**
C 07 C 143/00, C 07 C 143/22

(21) Application number: **85200257.5**

(22) Date of filing: **25.02.85**

(54) **Menthene sulphonates and the hydrogenated derivatives thereof.**

(30) Priority: **27.02.84 US 583557**
**27.02.84 US 584184**

(43) Date of publication of application:
**18.09.85 Bulletin 85/38**

(45) Publication of the grant of the patent:
**27.04.88 Bulletin 88/17**

(84) Designated Contracting States:
**AT BE CH DE FR GB IT LI NL SE**

(56) References cited:
**US-A-4 137 257**
**US-A-4 224 240**

(73) Proprietor: **UNILEVER NV**
**Burgemeester s'Jacobplein 1 P.O. Box 760**
**NL-3000 DK Rotterdam (NL)**
(84) **BE CH DE FR IT LI**

(73) Proprietor: **UNILEVER PLC**
**Unilever House Blackfriars P.O. Box 68**
**London EC4P 4BQ (GB)**
(84) **GB**

(72) Inventor: **Lamberti, Vincent**
**11 Holly Drive**
**Upper Saddle River New Jersey 07458 (US)**
Inventor: **Gutierrez, Eddie Nelson**
**216 Bellemeade Avenue**
**Fort Lee New Jersey 07024 (US)**

(74) Representative: **Waldren, Robin Michael et al**
**Unilever PLC Patent Division P.O. Box 68**
**Unilever House Blackfriars**
**London EC4P 4BQ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 155 040 B1

**Description**

The invention relates to novel menthene sulphonates and the hydrogenated derivatives thereof, their use as hydrotropes and processes for their preparation.

Many aqueous compositions contain organic components of poor water solubility. Hydrotropes are formulated into these liquids to increase the aqueous solubility of the hydrophobic organic components. Commonly employed hydrotropes include the salts of toluene, xylene or cumene sulphonates. While these commercial compounds perform satisfactorily, there is a need for lower cost alternatives, especially materials not derived from petrochemical feedstocks.

Among the relatively low-cost renewable raw materials is turpentine, an extract of pine trees. Major components of turpentine are α- and β-pinenes. When reacted with pyrosulphuryl chloride, α- and β-pinenes yield sulphonate compounds as in US Patent 2,220,678. Traynor et al, J. Org. Chem. Vol. 44 1557, 1979, reports that sodium p-menth-6-ene-2-sulphonate (I) can be formed from the dehydration of the reaction product between sodium sulphite and the α-pinene derivative limonene oxide. This publication further discloses the β-pinene will react with sodium bisulphite to form sodium p-menth-1-ene-7-sulphonate (II). The saturated analogs of I and II have apparently not yet been reported. Little is known concerning the utility of these sulphonated pinene derivatives. US Patents 4,224,240 and 4,283,347, however, mention the possible utility of p-menth-1-ene-7-sulphonate salts as detergents and surfactants.

It is now an object of this invention to describe novel menthene sulphonate and menthane sulphonate salts.

It is a further object to provide processes for their manufacture.

Another object of this invention is to provide a method for solubilizing only partially water-soluble compounds in aqueous formulations by means of menthene sulphonate and menthane sulphonate hydrotropes.

Accordingly, the present invention provides the use of p-menthene sulphonic acid derivatives and the alkali metal, alkaline earth metal, ammonium and alkylolammonium salts thereof, characterised by the general structure of the acid form:

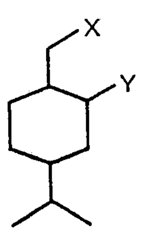

or

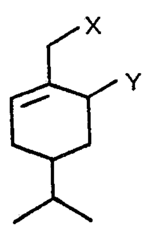

wherein X is H, $SO_3H$ or $CH_2OH$; wherein Y is H or $SO_3H$; wherein X is different from Y; as hydrotropes.

The compound p-menthene-6-sulphonic acid and its sodium salt are disclosed in US—A 4,137,257. However this acid and salt are not described as hydrotropes. Save for these, the compounds and salts according to the foregoing general formulae are believed to be novel. Thus, a further aspect of the present invention provides p-menthene sulphonic acid derivatives and the alkali metal, alkaline earth metal, ammonium and alkylolammonium salts thereof, characterised by the general structure of the acid form:

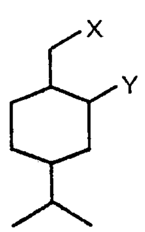

or

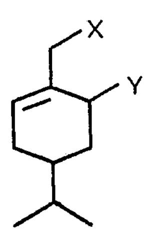

wherein X is H, $SO_3H$ or $CH_2OH$; wherein Y is H or $SO_3H$; wherein X is different from Y; other than p-menthene-6-sulphonic acid and its sodium salt.

p-Menthene-7-sulphonic acid, p-menthane-2-sulphonic acid, p-menthane-7-methylol-2-sulphonic acid and p-menth-6-ene-7-methylol-2-sulphonic acid and their alkali metal, alkaline earth metal, ammonium and alkylolammonium salts are hereby specifically disclosed.

A process is provided for preparing p-menth-6-ene-2-sulphonate salt which comprises reacting -pinene with a sulphite or bisulphite salt in the presence of a phase transfer catalyst, under free radical conditions, in an aqueous-organic co-solvent medium at a temperature of at least 40°C until said p-menth-6-ene-2-sulphonate salt is formed.

Furthermore, a process is provided for preparing the ammonium salts of p-menth-6-ene-7-methylol-2-sulphonic and p-menthane-7-methylol-2-sulphonic acids comprising:

(a) adding ammonium bisulphite to a stirred aqueous or an aqueous-organic co-solvent dispersion of

Nopol, the pH being maintained above 5.5;

(b) maintaining a temperature of at least about 40°C until said product has formed; and

(c) removing solvent from the reaction mixture; and

(d) optionally, hydrogenating the resultant products.

The step (a) will form predominately either the trans or cis isomers respectively. Preferably the pH is also maintained below 5.3.

Finally, a method is disclosed for increasing the solubility of an only partially water-soluble material comprising combining with said material in water a hydrotrope selected from the group consisting of the alkali metal, alkaline earth metal, ammonium and alkylolammonium salts of p-menth-6-ene-7-methylol-2-sulphonic acid, p-menth-6-ene-2-sulphonic acid, p-menth-1-ene-7-sulphonic acid and their saturated derivatives.

Salts of six menthane sulphonates and menthene sulphonates have been prepared and found to be effective hydrotropes in aqueous media. These comounds are identified by the following structural formulae:

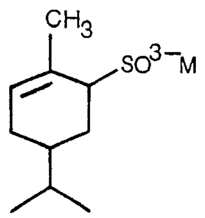

I

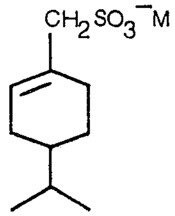

II

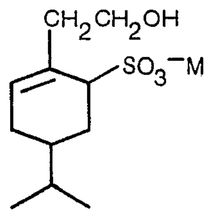

III

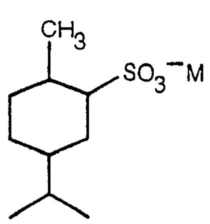

IV

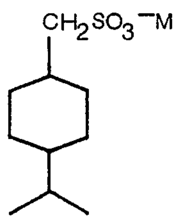

V

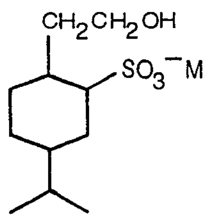

VI

where M is an alkali metal, alkaline earth metal, ammonium or alkylolammonium cation.

According to Traynor et al. in the J. Org. Chem. article, compound I could not, by sulphonation, be prepared directly from α-pinene. Competitive acid isomerization and hydration to α-terpineol was said to occur. These investigators were forced to prepare compound I by the circuitous route of epoxidizing α-pinene. The epoxide was then reacted with sulphite, acidified by ion-exchange chromatography and dehydrated to obtain I.

Now it has been found that compound I can be directly prepared from α-pinene at atmospheric pressure. The procedure involves incremental additions of bisulphite or sulphite to aqeuous or mixed aqueous-organic co-solvent, pinene solutions in the presence of air or a free radical initiator, ideally in the presence of a phase transfer catalyst. This is preferably a quaternary ammonium or phosphonium salt having at least one organic hydrophobic group.

Compound III is synthesized from 6,6-dimethylbicyclo-[3.1.1]hept-2-ene-2-ethanol (Nopol). Nopol is derived from a Prins reaction between β-pinene and formaldehyde. The procedure involves incremental additions of alkali metal or ammonium bisulphites to Nopol suspended in aqueous or mixed aqueous-organic co-solvent solutions in the presence of air or a free radical initiator.

Ammonium sulphite may be used by decomposition *in situ* as a source of bisulphite. In general, the optimum pH for these reactions is between about 5 and 6.

Where the ammonium salts of sulphite or bisulphite are employed, oxygen is the preferable free radical initiator. These reactions readily occur at atmospheric pressure.

Sodium sulphite or bisulphite reactions are preferably initiated by organic or inorganic peroxides. Representative of the former type of free radical initiators are tert-butyl peroxide, benzoyl peroxide, cumene hydroperoxide, tetralin hydroperoxide, isopropylbenzene hydroperoxide, acetyl peroxide, urea peroxide, diisopropyl peroxy dicarbonate, and, preferably, tert-butyl peroxy benzonate. Inorganic initiators such as hydrogen peroxide, hydrazine sulphate, sodium percarbonate and sodium persulphate are also useful. Organic diazo initiators, such as azobisisovaleronitrile and azobisisobutyronitrile, may similarly be employed. The free radical initiators are preferably combined with the sulphite or bisulphite and incrementally added to the Nopol (preparation of I) or pinene (preparation of III). Generally, from about 0.1 to about 10 mole %, based on moles Nopol or pinene, of the free radical initiator is used in the reaction mixture. Additionally, ultraviolet radiation may serve to establish the free radical conditions, including when an ultraviolet photo-initiator is added to the reaction mixture.

Although water can be used as the exclusive solvent, mixed water-organic co-solvent systems are

preferred. The organic co-solvents should be non-reactive in the process. Such solvents include alcohols, ethers, glycol ethers, esters, glycols, amines, amino alcohols and mixtures thereof. A combination of water with isopropanol or ethanol is preferred. Mixed aqueous-organic co-solvent systems may be combined in ratios ranging from 100:1 to 1:100. Preferably, the ratio of water to co-solvent should range from about 1:4 to 1:1. Water is present to assist the solubilization of the sulphite or bisulphite salt. Organic co-solvent is present for solubilizing the Nopol or pinene. The amount of solvent, either water, organic co-solvent or mixtures thereof, relative to Nopol will range from 100:1 to 1:100, respectively.

Reaction temperatures should range from at least 40°C to about 300°C. Preferably, the range should be from about 80°C to 150°C.

Relative molar ratios of sulphite or bisulphite to Nopol or pinene can range broadly from about 2:1 to 0.8:1. Preferably, their relative amounts should range from about 0.95 to 1.4:1.

In the case of the reaction with pinene, ammonium sulphite provides better yields than alkali metal sulphites or bisulphites. Ammonium bisulphite further improves yields and permits reaction to occur at lower temperatures, i.e. 50—50°C. Aqueous 45% ammonium bisulphite solutions may be utilized at the commercially available pH of 5.0—5.2 or adjusted to pH 5.6—6.0 with ammonia. Upon completion of the reaction, solvent is removed. In the α-pinene reaction, a crystalline monohydrate of the trans isomer of compound I is isolated. Some cis isomer is separated as a resinous dark yellow material. With ammonium bisulphite solutions of pH above 5.5, the trans isomer is formed in high specific purity. Trace amounts of cis isomer form at pH 5.0—5.3. The cis-sulphonate behaves differently from the trans, the former being susceptible to auto-oxidation.

Ammonium bisulphite provides better yields than ammonium sulphite, i.e., 28% versus 91% yield. Aqueous 45% ammonium bisulphite solutions may be utilized at the commercially available pH of 5.0—5.2 or adjusted to pH 5.6—6.0 with ammonia. On completion of the reaction, solvent is removed. At pH above 5.5, ammonium bisulphite reacts stereospecifically with Nopol to form the trans isomer of III. Lowering the pH to 5.3 or below affords cis isomer in significant amounts. Cis isomer separates as a resinous dark yellow material. The trans compound is crystalline. Furthermore, the cis isomer appears to be more susceptible to auto-oxidation. Hydrogen peroxide rapidly attacks cis isomer while the trans form is unaffected.

Compounds I, II and III can be converted to their saturated analogs IV, V and VI through hydrogenation. A variety of hydrogen methods and catalysts can be employed. Both soluble and heterogeneous catalysts are suitable. Among the heterogeneous variety are included platinum, palladium, rhodium, ruthenium, iridium and nickel, each metal being supported on suitable substrates to facilitate in the uptake of gaseous hydrogen.

In the hydrogenation of compounds I and II, 5% palladium on charcoal is preferred. With this catalyst, hydrogen is preferably held at about 6.89 bars (100 psi). Temperature is maintained around 80°C over a 1—5 hour period.

In certain instances, to save expensive catalyst, it becomes advisable to remove sulphur poisons by pre-treatment with Raney nickel. Pre-treatment involves stirring I or II with Raney nickel in water at 45—50°C.

Hydrogenation of III proceeds best at elevated pressures and temperatures. Raney nickel is the catalyst of choice. Temperatures in excess of 100°C and pressures of 500 34.47 bars (psi) hydrogen and above are preferred operating conditions.

Ammonium and alkylolammonium salts of IV and V may be obtained by treatment of the corresponding hydrogenated sodium salt by passage through an ion-exchange column and neutralization of the liberated sulphonic acid with the appropriate base (e.g. ammonium hydroxide or alkylolamines such as ethanolamine, diethanolamine and triethanolamine).

Sodium, potassium, alkaline earth meal and alkylolammonium salts of III and VI may be obtained by treatment of the corresponding ammonium salt by passage through an ion-exchange column and neutralization of the liberated sulphonic acid with the appropriate base (e.g. sodium hydroxide, potassium hydroxide and alkylolamines such as monoethanolamine, diethanolamine and triethanolamine).

Sulphonates IV, V and VI have better storage stability than their unsaturated precursors. Upon prolonged storage, compounds I, II and III develop a yellow colour.

Compounds I through VI are here shown to be effective hydrotropes for solubilizing only partially water-soluble materials into aqueous systems. Hydrotropes are commercially important, in particular, as components in aqueous cleaning compositions. These compositions frequently contain surfactants such as anionic, nonionic cationic, zwitterionic or amphoteric actives or mixtures thereof. These surfactants are set forth in "Surface Active Agents and Detergents" by Schwartz, Perry & Berch, Vol. II, Interscience Publishers, Inc., 1958, herein incorporated by reference. These surfactants are generally employed at from 1% to 50% by weight of the total cleaning formulation.

Stability Performance Evaluation

A measure of the effectiveness of a hydrotrope is the amount required to stabilize a liquid composition undergoing freeze-thaw cycling.

The procedure for evaluating freeze-thaw stability involves subjecting a sample in a glass jar to six controlled freeze-thaw cycles between −17.8°C (0°F) and 21.1°C (70°F). Typically, inspection of samples is performed after each 1, 2, 3 and 6 cycles. Cycling time between −17.8°C (0°F) and 21.1°C (70°C) is 24 hours,

except over weekends when temperature is maintained at 21.1°C (70°F) for 48 hours. Six hours are necessary for the temperature in the room to drop from 21.1°C (70°F) to −17.8°C (0°F) and 4 hours to rise from −17.8°C (0°F) to 21.1°C (70°F). These cycles are thought to simulate the most extreme conditions for storage and transportation of hydrotrope-containing commercial products during winter months.

The following examples will more fully illustrate the emodiments of this invention. All parts, percentages and proportions referred to herein and in the appended claims are by weight unless otherwise indicated.

EXAMPLE 1

A typical light-duty liquid dishwashing formulation is outlined in Table I. In this base formulation where incorporated the various hydrotropes of this invention.

TABLE I
Light-Duty Liquid Detergent Base Composition

| Components | % Actives by Weight in Formula: Formula A | Formula B |
|---|---|---|
| Ammonium linear $C_{10}$—$C_{15}$ alkylbenzene sulphonate | 24 | 30 |
| Ammonium linear $C_{12}$—$C_{14}$ alcohol triethoxysulphate | 5 | 5 |
| Lauric diethanolamide | 3 | — |
| Hydrotrope* | — | — |
| Magnesium chloride | — | 0.5 |
| Water | to 100 | to 100 |

* Identity and amounts as per following Examples.

TABLE II
Heavy-Duty Built Liquid Laundry Detergent

| Components | % Active by Weight in Formula |
|---|---|
| Sodium linear $C_{10}$—$C_{15}$ alkylbenzene sulphonate | 17 |
| Sodium nitrilotriacetic acid | 13 |
| Hydrotrope* | — |
| Water | to 100 |

* Identity and amounts as per following Examples.

EXAMPLE 2

Unsaturated and saturated sulphonates III and VI were separately incorporated as a hydrotrope in the base aqueous liquid detergent formula A. Several concentration levels were evaluated. Results are recorded in Table III. Data therein indicate that 6% of either the unsaturated or saturated sulphonated Nopol is sufficient to provide freeze-thaw stability toward formula A. Compounds III and VI are more efficient as hydrotropes than the reference, ammonium xylene sulphonate.

TABLE III
Freeze-Thaw Stability of Sulphonated Nopol Derivatives

| Sample Number | Hydrotrope* | Concentration (% Weight) | Freeze-Thaw Stability 6 cycles at 0—70°F |
|---|---|---|---|
| 1 | Ammonium xylene sulphonate (Control) | 8 | Stable |
| 2 | Ammonium xylene sulphonate (Control) | 6 | Two Phases |
| 3 | Compound III | 4 | Two Phases |
| 4 | Compound III | 6 | Stable |
| 5 | Compound III | 8 | Stable |
| 6 | Compound VI | 6 | Stable |
| 7 | Compound VI | 7 | Stable |
| 8 | Compound VI | 8 | Stable |
| 9 | Compound VI | 9 | Stable |

* Compounds III and VI evaluated as the ammonium salt.

EXAMPLE 3

Compounds I, II, IV and VI were evaluated for their efficiency as hydrotropes in freeze-thaw stability tests. These compounds were incorporated in the base formulation A of Table I at several concentrations to determine the minimum amount of hydrotrope needed to provide adequate stability. Ammonium xylene sulphonate served as the reference hydrotrope.

TABLE IV
Freeze-Thaw Stability Performance

| Formulation | Hydrotrope | Concentration (% Weight) | Freeze-Thaw Stability (6 cycles at 0—70°F) |
|---|---|---|---|
| 1 Control | Ammonium xylene sulphonate | 8 | Stable |
| 2 | Sodium salt of I | 8 | Stable |
| 3 | Ammonium salt of I | 8 | Stable |
| 4 | Sodium salt of IV | 8 | 10—15% gel |
| 5 | Ammonium salt of IV | 8 | 20% gel |
| 6 | Ammonium salt of IV | 12 | 12% gel |
| 7 | Sodium salt of II | 8 | 10% gel on top |
| 8 | Ammonium salt of II | 8 | 8% gel on top |
| 9 | Ammonium salt of II | 9 | 10% gel on top |
| 10 | Ammonium salt of II | 10 | thin film of gel |
| 11 | Ammonium salt of II | 12 | Stable |
| 12 | Sodium salt of V | 8 | 10—15% gel |
| 13 | Ammonium salt of V | 8 | 15% gel |
| 14 | Ammonium salt of V | 10 | 3—5% gel |
| 15 | Ammonium salt of V | 12 | Stable |

Table IV indicates that compounds I, II, IV and V all display hydrotrope properties. They approach ammonium xylene sulphonate in performance. At the 12% level, compound II and its saturated analog V provided stable liquids under freeze-thaw conditions. Compound I provided equivalent stability to that of the control at 8% active concentration. The saturated analog IV was less effective.

EXAMPLE 4

This example illustrates the freeze-thaw stability of compounds III and VI incorporated in base formula B, Table I. Table V tabulates the hydrotrope performance. Ammonium xylene sulphonate in formula B is a more efficient hydrotrope than III.

TABLE V
Freeze-Thaw Stability Performance of Unsaturated Sulphonated Nopol

| Sample Number | Hydrotrope* | Concentration (% Weight) | Freeze-Thaw Stability (6 cycles at 0—70°F) |
|---|---|---|---|
| 1 | Ammonium xylene sulphonate (Control) | 9 | Clear |
| 2 | Compound III | 6 | Two Phases |
| 3 | Compound III | 7 | Two Phases |
| 4 | Compound III | 8 | Two Phases |
| 5 | Compound III | 9 | Two Phases |

* Compound III evaluated as the ammonium salt.

EXAMPLE 5

An illustration of hydrotropic performance of compound III (ammonium salt) in a built heavy duty liquid composition is herein provided. The data in Table VI indicate that III is a more efficient hydrotrope than sodium xylene sulphonate. It is also apparent that mixtures of sodium xylene sulphonate with III interact to provide unexpectedly superior results. The compositions evaluated were in accordance with the formula outlined in Table II.

TABLE VI

| Sample | Hydrotrope (% weight) | Physical Stability |
|---|---|---|
| 1 | Sodium xylene sulphonate (8%) | 20% separated top |
| 2 | Sodium xylene sulphonate (10%) | 15% separated top |
| 3 | Sodium xylene sulphonate (8%) Compound III (1%) | Clear with schlieren |
| 4 | Sodium xylene sulphonate (8%) Compound III (2%) | Traces of separated top |
| 5 | Compound III (6%) | Clear with schlieren |
| 6 | Compound III (8%) | Trace of separated bottom |

EXAMPLE 6

Comparisons between compound III (sodium salt) and sodium xylene sulphonate in the formulation of Table VI are detailed in Table VII. Approximately 9% of III was required to achieve stability at room temperature. Sodium xylene sulphonate was required only at 6% to achieve stability at the same temperature.

TABLE VII

| Hydrotrope (Weight %) | Stability: Room Temperature | Stability: 35°F overnight | Stability: Freeze-Thaw (0—70°F) 6 cycles |
|---|---|---|---|
| Sodium xylene sulphonate (6%) | Clear | Two Phases | — |
| Sodium xylene sulphonate (8%) | Clear | Clear | About 8% gel on top |
| Compound III (8%) | Two Phases | Two Phases | — |
| Compound III (9%) | Clear | Two Phases | — |
| Compound III (10%) | Clear | Clear | Stable (Clear) |

EXAMPLE 7

Preparation of (−) Sodium (2S, 4R) p-menth-6-ene-2 sulphonate (I)

Method (1)

In a 2 litre, 3-neck Morton flask equipped with a stirrer and reflux condenser, 136 g (1 mole) α-pinene and one litre isopropanol:water in the ratio 1:1 were brought to reflux. Sodium bisulphite, 110 grams (1.06 mole) was added at the rate of 26 grams per hour along with several additions of 2—3 drops t-butyl peroxybenzoate. The solution was refluxed for 10 hours.

Thereafter, the solution was evaporated to dryness and the residue extracted with hot 3A ethanol. Extraction was done three times with 500 ml solvent each time. The combined extracts were evaporated and the residual solids were then dried *in vacuo* over phosphorus pentoxide. A yield of 6% was obtained. The NMR spectrum exhibited the following peaks: $CH_3$ (doublet. 0.75 and 0.86δ); $CH_3$ (singlet, 1.84δ); $CH_2$ and CH (multiplet, 1.00—2.40δ); CH (multiplet, 3.23—3.50δ); and CH (multiplet, 5.00—5.26δ).

Method (2)

In a 1-litre, 3-neck Morton flask, 130 g of α-pinene and 10 g of t-benzyltrimethyl ammonium hydroxide were dissolved in 400 ml of 1:1 isopropanol:water and brought to reflux. Sodium bisulphite, 120 g (1.15 mole) was added at the rate of 10 grams per hour along with several additions of 2—3 drops of t-butyl peroxybenzoate. The solution was heated for 14 hours. The solvents were then removed by distillation. The residue was extracted with 3A ethanol. Extracts were combined and evaporated to dryness. Residues were dried *in vacuo* over phosphorus pentoxide. Crude product in the amount of 130 grams was obtained. Karl Fisher analysis indicated the presence of 2.9% water. NMR indicated 58% purity.

EXAMPLE 8

Preparation of (−) Ammonium (2S, 4R) p-menth-6-ene-2-sulphonate (I)

Ammonium Sulphite Method

In a 3-litre flask equipped with stirrer, 136 g (1.0 mole) α-pinene was dissolved in 800 g isopropanol and 200 g water. The solution was brought to reflux. Ammonium sulphite monohydrate, 132 g (0.97 mole), was added at the rate of about 0.5 grams per minute. This solution was then refluxed for 15 hours. Thereafter, the solution was filtered, evaporated to dryness *in vacuo* and dried over phosphorus pentoxide. A product weighing 179 grams was obtained. NMR analysis indicated 81% purity (62% yield). Karl Fisher analysis showed 1.6% water present.

EXAMPLE 9

Preparation of (−) Ammonium (2S, 4R) p-menth-6-ene-2-sulphonate (I)

Ammonium Bisulphite Method

In a 500 ml flask equipped with a magnetic stirrer were placed 55 g α-pinene, 100 ml isopropanol, 150 ml water and 2 g (60%) benzyltrimethyl ammonium chloride. While stirring, 120 g (45%) ammonium bisulphite was gradually added. The mixture was stirred for 24 hours at 40—45°C. Solvents were then removed by distillation *in vacuo*. The residue was dissolved in 300 ml isopropanol. The isopropanol solution was filtered and evaporated to dryness. A product weighing 93 grams was isolated. Its purity was 82.5%, corresponding to an 81% yield.

EXAMPLE 10

Preparation of (−) Sodium (4S)-p-menth-1-ene-7-sulphonate (II)

Experiment (1)

A mixture of 140 g (1.03 moles) β-pinene and 650 ml water was heated to reflux in a 2-litre, 3-necked flask equipped with stirrer and reflux condenser. Then, 125 g (1.2 moles) sodium bisulphite was added in 25 gram increments per hour along with several additions of 2—3 drops of t-butyl peroxybenzoate. Heat was applied to the mixture for a period of 7.5 hours. The solution was allowed to stand overnight. Crystals which had formed were filtered off and dried *in vacuo* over phosphorus pentoxide. A total of 89 grams was collected, having 91% purity (NMR analysis), indicating a 32% yield. The NMR spectrum of the compound included signals at: $CH_3$ (doublet, 0.75 and 0.85δ); CH and $CH_2$ (multiplets, 1.00—2.40δ); $CH_2$ (singlet, 3.40δ)) and CH (broad singlet, 5.80—5.90δ).

Experiment (2)

In a 1-litre, 3-neck flask, 70 grams (0.51 mole) β-pinene and 325 ml water were heated to reflux. Sodium bisulphite, 70 grams, was added at the rate of 13 grams per hour. The solution was refluxed for 7 hours and allowed to stand overnight.

Excess β-pinene, 3.8 grams, was removed by distillation along with 100 ml of water. Upon cooling the solution, 106 grams (95% pure) of product was crystallized. The solution was evaporated to dryness and residue extracted with 300 ml hot ethanol. Ethanol was removed by distillation and residual solvent evaporated *in vacuo*. The product remained as a residue of 6.7 grams weight and 21.2% purity. Total yield was 87%.

EXAMPLE 11

Preparation of (−) Ammonium (4S)-p-menth-1-ene-7-sulphonate (II)

Ammonium Sulphite Method

A solution of 136 g (1 mole) β-pinene in 600 g isopropanol and 200 g water was heated to reflux in a 2-litre, 3-neck Morton flask. Ammonium sulphite monohydrate, 132 g (0.99 mole), was added at the rate of about 0.5 grams per minute. The solution was refluxed for a total of 15 hours. Thereafter, the solution was filtered, the filtrate being evaporated to dryness. There was obtained 156 grams product having 72.1% purity (47% yield).

The solid product was redissolved in 3A ethanol. Solvent-insoluble residues were filtered off. Filtrate solvent was evaporated to dryness. Residue product was dried *in vacuo* over phosphorus pentoxide. A product was obtained having 74.8% purity by NMR analysis. Karl Fisher titration indicated 1.5% water.

EXAMPLE 12

Preparation of (−) Ammonium (4S)-p-menth-1-ene-7-sulphonate (II)

Ammonium Bisulphite Method

In a 1-litre flask equipped with magnetic stirrer were placed 450 ml isopropanol, 240 ml water and 54.4 grams (0.4 mole) β-pinene. Ammonium bisulphite, 105 grams (45% O), was then added thereto. The mixture was stirred at 40—45°C for 22 hours. Thereafter, the solution was evaporated to dryness. Residue was extracted with methanol. The methanolic solution was filtered, and the resultant filtrate evaporated to dryness. Obtained were 84 grams product (85.8% purity; 77% yield).

EXAMPLE 13

Preparation of Sodium-p-menthane-2-sulphonate (IV)

Sodium p-menth-6-ene-2-sulphonate (51.2 g) as prepared in Method 2 was dissolved in 500 ml water and mixed with 22.08 grams of Raney nickel catalyst at 45—50°C. Sulphur poisons were removed by this pre-treatment.

After 2 hours, the mixture was filtered and transferred to a 1-litre Parr bomb. Twelve grams of 5% palladium on carbon were added to the mixture. The bomb was sealed and flushed several times with hydrogen. Hydrogenation was performed at 75—80°C for 3—4 hours at 100 psi. Thereafter, the bomb was cooled, opened and the solution filtered. Filtrate was evaporated to dryness and the residue stored over phosphorus pentoxide. The product, weighing 42.4 grams, was 95.4% pure by NMR analysis. The NMR spectrum exhibited signals at: $CH_3$ (doublet, 1.04 and 1.12δ); and $CH_2$ and CH (multiplet, 1.20—2.50δ); $CH_3$ (multiplet, 1.60δ); and CH (multiplet, 3.00—3.20δ).

EXAMPLE 14

Preparation of Ammonium p-menthane-2-sulphonate (IV)

Sodium-p-menthane-2-sulphonate, 35 grams, prepared as in Example 13, was passed through an ion-exchange column. The eluate containing p-menthane-2-sulphonic acid was neutralized with dilute ammonium hydroxide. The solution was evaporated to dryness and the residue stored *in vacuo* over phosphorus pentoxide. The product was 91.7% pure by NMR analysis.

EXAMPLE 15

Preparation of Sodium (4S)-p-menthane-7-sulphonate (V)

Sodium (4S)-p-menth-1-ene-7-sulphonate, 75 grams, was dissolved in 500 ml of water. The solution was then placed in a 1-litre Parr Reactor together with 12 grams (5%) palladium on carbon. The Parr Reactor was sealed and flushed with hydrogen gas several times to remove oxygen. Hydrogen was charged to the bomb at 100 psi and the contents heated to 75—80°C for 2 hours. Subsequently, the bomb was cooled, opened and the solution filtered to remove catalyst. Solvent was evaporated from the solution and the residue dried *in vacuo* over phosphorus. Seventy grams of product with NMR purity of 99.3% was obtained. The NMR spectrum exhibited signals at: $CH_3$ (doublet, 1.00 and 1.14δ); CH and $CH_2$ (multiplet, 1.00—1.30δ); and $CH_2$ (triplet, centred at 3.07δ).

EXAMPLE 16

Preparation of Ammonium (4S)-p-menthane-7-sulphonate (V)

An aqueous solution of 30 grams sodium (4S)-p-menthane-7-sulphonate, prepared according to Example 15, was passed through a Permutit Q101 cationic exchange column to remove sodium ions. The eluate containing (4S)-p-menthane-7-sulphonic acid was neutralized with ammonium hydroxide solution. The resultant residue was dried over phosphorus pentoxide. Obtained were 29 grams product of 99.9% purity.

EXAMPLE 17

Preparation of Ammonium p-menth-6-ene-7-methylol-2-trans-sulphonate (III)

Ammonium Sulphite Method

In a 2-litre, 3-neck flask equipped with stirrer and reflux were placed 600 grams isopropanol, 200 grams water and 166 grams (1 mole) Nopol. To the refluxing mixture were added 134 grams aqueous ammonium sulphite (1 mole) at the rate of 13.4 grams per hour. The solution was refluxed for a total of 14 hours. Then it was evaporated to dryness. The residue was dissolved in isopropanol, filtered and the resulting filtrate evaporated to dryness. A product weighing 102 grams (74% active) was obtained in 28% yield.

EXAMPLE 18

Preparation of Ammonium-p-menth-6-ene-7-methylol-2-trans-sulphonate (III)

Ammonium Bisulphite Method 1

In a 500 ml flask, equipped with a magnetic stirrer, were placed 66.4 grams (0.4 mole) Nopol, 100 ml ethanol and 100 ml water. To the stirred mixture was added 110 grams (45%) ammonium bisulphite solution adjusted with ammonia to pH 5.6 to 6.0. The mixture was stirred at 40—45°C for 24 hours.

Solvents were distilled off and the residue extracted with 300 ml ether. About one gram of Nopol was recovered. The residue was then dissolved in 300 ml isopropanol. Insoluble materials weighing 7.5 grams

were removed by filtration. Isopropanol was removed by vacuum distillation, leaving a residue which was further dried over phosphorus pentoxide. A product weighing 109 grams (88.5% active) was obtained in 91% yield.

Ammonium Bisulphite Method 2

In a litre flask equipped with magnetic stirrer were placed 420 ml isopropanol, 240 ml water and 66 grams (0.4 mole) Nopol. While the solution was being stirred, 105 grams (45%) ammonium bisulphite solution (pH 5.6—6.0) was added. The solution was stirred for 24 hours. Solvents were evaporated *in vacuo* and residue dissolved in methanol. The methanol solution was filtered to remove inorganics. Filtrate was evaporated to give 102 grams residue. The residue was dissolved in hot isopropanol. Recrystallization gave 94 grams (99.2% purity) of a crystalline product, indicating a yield of 88.7%). The NMR analysis exhibited the following spectrum: $CH_3$ (doublet, 0.75—0.90δ, coupling constant 6 Hz); $CH_2$ (multiplet, 1.20—2.70δ); CH (multiplet, 1.20—2.70δ); CH (multiplet, 3.45—3.75δ); $CH_2$ (multiplet centred at 3.73δ) and CH (multiplet, 5.72—6.00δ).

Ammonium Bisulphite Method 3

In a 1-litre, 3-neck Morton flask were placed 99.5 grams (0.6 mole) Nopol, 125 ml ethanol and 50 ml water. The solution was de-aerated with nitrogen gas, using a gas dispersion tube. The solution was heated to 70°C with vigorous agitation.

In an addition funnel were combined at 10°C, 110 grams (0.5 mole) 45% ammonium bisulphite solution and 17.6 grams concentrated ammonia. This solution was brought to room temperature and added slowly to the contents of the Morton flask with concurrent addition of 1.38 grams (8.4 mmol) azobisisovaleronitrile in 25 ml aqueous ethanol. Addition was complete in 45 minutes. Thereafter, the mixture was stirred a further 6.5 hours at 70°C.

The above solution was steam-distilled to remove Nopol. Thereafter, the remaining aqueous solution was evaporated to dryness, leaving behind the product. This material weighed 124.8 grams (70% purity), corresponding to a 66% yield.

EXAMPLE 19

Preparation of Ammonium p-meth-6-ene-7-methylol-2-cis-sulphonate (III)

In a litre flask equipped with a magnetic stirrer were placed 150 ml ethanol and 83 grams Nopol (0.5 mole). To this solution was added 156 grams (45%; 0.7 mole) ammonium bisulphite having pH 5.0—5.1. The mixture was stirred at 40°C for 24 hours.

Ammonium hydroxide, 16 grams, was added to the reactor, bringing the pH to 6.8—7.0. Ethanol was distilled and the aqueous solution extracted three times with 200 ml chloroform. The aqueous solution was evaporated to a heavy syrup. Methanol, 350 ml, was added. The solution was allowed to stand for 30 minutes and filtered to remove inorganics. Methanol was evaporated from the solution, affording a syrup that was dried over phosphorus pentoxide. A product weighing 134 grams was obtained. Unreacted Nopol, 18 grams, was recovered from the chloroform solution. The product was stored under nitrogen to prevent oxidation. NMR analysis indicated product purity of 72.6% (57.3% yield). The NMR spectrum of the product was as follows: $CH_3$ (doublet, 0.80—0.92δ, coupling constant 2.1 Hz); $CH_2$ (multiplet, 1.2—2.60δ); CH (multiplet, 1.20—2.60δ); CH (multiplet, 3.45—3.70δ); $CH_2$ (multiplet centred at 3.45—3.75δ) and CH (multiplet, 5.65—5. 90δ).

EXAMPLE 20

Preparation of Ammonium p-menthane-7-methylol-2-sulphonate (VI)

Seventy grams of ammonium p-menth-6-ene-7-methylol-2-sulphonate prepared as in Example 18 (Method 2) was stored overnight at 45—50°C over 500 ml water containing 50 grams Raney nickel catalyst. The mixture was transferred to a one-litre Parr bomb. Raney nickel, 12 grams, was added to the mixture. The bomb was sealed and flushed several times with hydrogen. Hydrogenation was performed at 125°C under 500 psig of hydrogen for a period of 4 hours.

The mixture was removed from the bomb, filtered free of catalyst and allowed to stand. A very small amount of light brown solid, which crystallized out, was filtered before evaporating the filtrate to dryness. A total of 65 grams (100% purity) was obtained. The NMR spectrum consisted of: $CH_3$ (doublet, 0.80—1.00δ); $CH_2$ (multiplet, 1.00—2.30δ); CH (multiplet, 1.00—2.30δ); CH (multiplet, 2.94—3.20δ) and $CH_2$ (triplet centred at 3.57δ).

**Claims**

1. The use of p-menthene sulphonic acid derivatives and the alkali metal, alkaline earth metal, ammonium and alkylolammonium salts thereof, characterised by the general structure of the acid form:

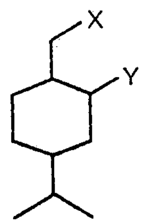

or

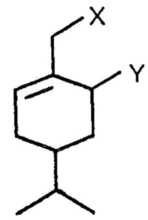

wherein X is H, $SO_3H$ or $CH_2OH$; wherein Y is H or $SO_3H$; wherein X is different from Y; as hydrotropes.

2. p-Menthene sulphonic acid derivatives and the alkali metal, alkaline earth metal, ammonium and alkylolammonium salts thereof, characterised by the general structure of the acid form:

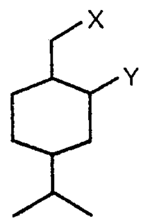

or

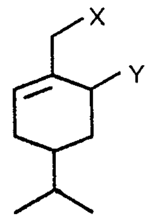

wherein X is H, $SO_3H$ or $CH_2OH$; wherein Y is H or $SO_3H$; wherein X is different from Y; other than p-menthene-6-sulphonic acid and its sodium salt.

3. A process for the preparation of the ammonium salt of p-menth-6-ene-7-methylol-2-trans-sulphonic acid, characterised in that it comprises:

(a) adding ammonium sulphite or bisulphite to a stirred aqueous or an aqueous-organic co-solvent dispersion of Nopol, the pH being maintained above 5.5;

(b) maintaining a temperature of at least about 40°C until said product has formed; and

(c) removing solvent from the reaction mixture.

4. A process for the preparation of the ammonium salt of p-menthane-7-methylol-2-trans-sulphonic acid, characterised in that it comprises:

(a) adding ammonium sulphite or bisulphite to a stirred aqueous or an aqueous-organic co-solvent dispersion of Nopol, the pH being maintained above 5.3;

(b) maintaining a temperature of at least about 40°C until said product has formed;

(c) removing solvent from the reaction mixture; and

(d) hydrogenating the resultant product.

5. A process for the preparation of the ammonium salt of p-menth-6-ene-7-methylol-2-cis-sulphonic acid, characterized in that it comprises:

(a) adding ammonium sulphite or bisulphite to a stirred aqueous or an aqueous-organic co-solvent dispersion of Nopol, the pH being maintained above 5.5;

(b) maintaining a temperature of at least about 40°C until said product has formed; and

(c) removing solvent from the reaction mixture.

6. A process for the preparation of the ammonium salt of p-menthane-7-methylol-2-cis-sulphonic acid, characterized in that it comprises:

(a) adding ammonium sulphite or bisulphite to a stirred aqueous or an aqueous-organic co-solvent dispersion of Nopol, the pH being maintained above 5.3;

(b) maintaining a temperature of at least about 40°C until said product has formed;

(c) removing solvent from the reaction mixture; and

(d) hydrogenating the resultant product.

7. A process for the preparation of a p-menth-6-ene-2-sulphonate salt, characterized in that it comprises reacting α-pinene with a sulphite or bi-sulphite salt in the presence of a phase-transfer catalyst, under free radical conditions, in an aqueous or an aqueous-organic co-solvent medium at a temperature of at least 40°C until said p-menth-6-ene-2-sulphonate salt is formed.

8. A process according to any of the claims 3 to 7, wherein the organic co-solvent is isopropanol or ethanol.

9. A process according to any of the claims 3 to 8, wherein the ratio of water to organic co-solvent ranges from 1:100 to 100:1.

10. A process according to any of the claims 3 to 9, wherein the reaction is catalyzed by free radicals generated by an organic or inorganic peroxide, azo compound or oxygen.

11. A process according to claim 7, wherein the phase-transfer catalyst is a quaternary ammonium or phosphonium salt having at least one organic hydrophobic group.

12. A process according to claim 7, wherein the peroxide is tert-butyl peroxybenzoate.

**Patentansprüche**

1. Verwendung von p-Menthensulfonsäure-derivaten und ihren Alkali-, Erdalkali-, Ammonium- und Alkylolammonium-Salzen, gekennzeichnet durch die allgemeine Struktur der Säureform:

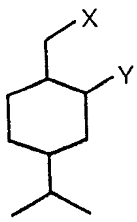

oder

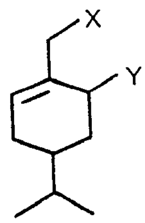

worin X H, $SO_3H$ oder $CH_2OH$ bedeutet, Y H oder $SO_3H$ bedeutet, wobei X unterschiedlich ist von Y; als Hydrotrope.

2. P-Menthensulfonsäure-derivate und ihre Alkali-, Erdalkali-, Ammonium- und Alkylolammonium-Salze, gekennzeichnet durch die allgemeine Struktur der Säureform

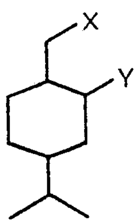

oder

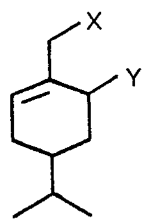

worin X H, $SO_3H$ oder $CH_2OH$ bedeutet, Y H oder $SO_3H$ bedeutet und X unterschiedlich von Y ist, mit Ausnahme von p-Menthen-6-sulfonsäure und ihrem Natriumsalz.

3. Verfahren zur Herstellung des Ammoniumsalzes von p-Menth-6-en-7-methylol-2-trans-sulfonsäure, dadurch gekennzeichnet, daß es umfaßt:

a) Zugabe von Ammoniumsulfit oder -bisulfit zu einer gerührten wässrigen oder einer wässrigen-organischen-co-solvens-Dispersion von Nopol, wobei der pH-Wert oberhalb 5,5 gehalten wird;

b) Aufrechterhaltung einer Temperatur von mindestens etwa 40°C bis das Produkt gebildet ist; und

c) Entfernung des Lösungsmittels aus der Reaktionsmischung.

4. Verfahren zur Herstellung des Ammoniumsalzes von p-Menthan-7-methylol-2-trans-sulfonsäure, dadurch gekennzeichnet, daß es umfaßt:

a) Zugeben von Ammoniumsulfit oder -bisulfit zu einer gerührten wässrigen oder einer wässrigen-organischen-co-solvens-Dispersion von Nopol, wobei der pH-Wert oberhalb 5,3 gehalten wird;

b) Aufrechterhalten einer Temperatur von mindestens etwa 40°C bis das Produkt gebildet ist;

c) Entfernung des Lösungsmittels aus der Reaktionsmischung; und

d) Hydrierung des entstandenen Produktes.

5. Verfahren zur Herstellung des Ammoniumsalzes von p-Menth-6-en-7-methylol-2-cis-sulfonsäure, dadurch gekennzeichnet, daß es umfaßt:

a) Zugeben von Ammoniumsulfit oder -bisulfit zu einer gerührten wässrigen oder einer wässrigen-organischen-co-solvens-Dispersion von Nopol, wobei der pH-Wert bei oder unterhalb 5,5 gehalten wird;

b) Aufrechterhalten einer Temperatur von mindestens etwa 40°C bis das Produkt gebildet ist; und

c) Entfernene des Lösungsmittels aus der Reaktionsmischung.

6. Verfahren zur Herstellung des Ammoniumsalzes von p-Menthan-7-methylol-2-cis-sulfonsäure, dadurch gekennzeichnet, daß es umfaßt:

a) Zugeben von Ammoniumsulfit oder -bisulfit zu einer gerührten wässrigen oder einer wässrigen-organischen-co-solvens-Dispersion von Nopol, wobei der pH-Wert oder unterhalb 5,3 gehalten wird;

b) Aufrechterhalten einer Temperatur von mindestens etwa 40°C bis das Produkt gebildet ist;

c) Entfernene des Lösungsmittels aus der Reaktionsmischung; und

d) Hydrieren des entstandenen Produktes.

7. Verfahren zur Herstellung eines p-Menth-6-en-2-sulfonatsalzes, dadurch gekennzeichnet, daß es umfaßt die Umsetzung von α-Pinen mit einem Sulfit oder Bisulfitsalz in Anwesenheit eines Phasentransferkatalysators unter Bedingungen der Bildung freier Radikale, in einem wässrigen oder einem wässrigen-organischen-co-solvens-Medium bei einer Temperatur von mindestens 40°C bis das p-Menth-6-en-2-sulfonatsalz gebildet ist.

8. Verfahren gemäß einem der Ansprüche 3 bis 7, worin das organische-co-solvens Isopropanol oder Ethanol ist.

9. Verfahren gemäß einem der Ansprüche 3 bis 8, worin das Verhältnis von Wasser zu organischem Co-solvens im Bereich von 1:100 bis 100:1 liegt.

10. Verfahren gemäß einem der Ansprüche 3 bis 9, worin die Reaktion katalysiert wird durch freie Radikale erzeugt durch ein organisches oder anorganisches Peroxyd, eine Azoverbindung oder Sauerstoff

11. Verfahren gemäß Anspruch 7, worin der Phasentransferkatalysator ein quaternäres Ammonium- oder Phosphonium-salz mit mindestens einer organischen hydrophoben Gruppe ist.

12. Verfahren gemäß Anspruch 7, worin das Peroxyd tert-Butyl Peroxybenzoat ist.

**Revendications**

1. Utilisation de dérivés d'acide p-menthène-sulfonique et de ses sels de métaux alcalins, de métaux alcalino-terreux d'ammonium et d'alkylammonium, caractérisés par la structure générale suivante de la forme acide:

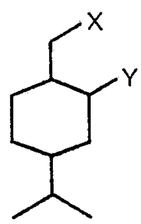

ou

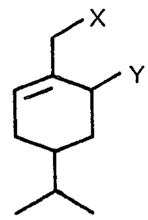

dans laquelle X représente H, $SO_3H$ ou $CH_2OH$, Y représente H ou $SO_3H$ et X est différent de Y, à titre d'hydrotropes.

2. Dérivés d'acide p-menthène-sulfonique et de ses sels de métaux alcalins, de métaux alcalino-terreux d'ammonium et d'alkylammonium, caractérisés par la structure générale suivante de la forme acide:

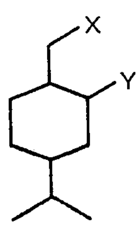

ou

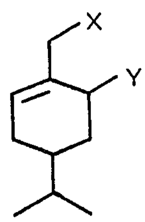

dans laquelle X représente H, $SO_3H$ ou $CH_2OH$, Y représente H ou $SO_3H$, X est différent de Y, autres qui l'acide p-menthène-6-sulfonique et son sel de sodium.

3. Procédé de préparation du sel d'ammonium de l'acide p-menth-6-ène-7-méthylol-2-trans-sulfonique, caractérisé en ce qu'il comprend les étapes consistant:

(a) à ajouter du sulfite ou bisulfite d'ammonium à une dispersion aqueuse agitée ou une dispersion aqueuse dans un co-solvant organique de Nopol, le pH étant maintenu au dessus de 5,5;

(b) à maintenir une température d'au moins 40°C jusqu'à formation dudit produit; et

(c) à éliminer le solvant du mélange de réaction.

4. Procédé de préparation du sel d'ammonium de l'acide p-menthane-7-méthylol-2-trans-sulfonique, caractérisé en ce qu'il comprend les étapes consistant:

(a) à ajouter du sulfite ou bisulfite d'ammonium à une dispersion aqueuse agitée ou une dispersion aqueuse avec un cosolvant organique de Nopol, le pH étant maintenu au dessus de 5,3;

(b) à maintenir une température d'au moins 40°C environ jusqu'à formation dudit produit;

(c) à soutirer le solvant du mélange de réaction; et

(d) à hydrogéner le produit résultant.

5. Procédé de préparation du sel d'ammonium de l'acide p-menth-6-ène-7-méthylol-2-cis-sulfonique, caractérisé en ce qu'il comprend les étapes consistant:

(a) à ajouter du sulfite ou bisulfite d'ammonium à une dispersion aqueuse agitée ou dispersion aqueuse avec cosolvant organique de Nopol, en maintenant le pH à 5,5 ou plus bas;

(b) à maintenir une température d'au moins 40°C environ jusqu'à formation dudit produit; et

(c) à soutirer le solvant du mélange de réaction.

6. Procédé de préparation du sel d'ammonium de l'acide p-menthane-7-méthylol-2-cis-sulfonique, caractérisé en ce qu'il comprend les étapes consistant:

(a) à ajouter du sulfite ou bisulfite d'ammonium à une dispersion aqueuse agitée ou une dispersion aqueuse avec cosolvant organique de Nopol, en maintenant le pH à 5,3 ou au dessous;

(b) à maintenir une température d'au moins 40°C jusqu'à formation dudit produit;

(c) à éliminer le solvant du mélange de réaction; et

(d) à hydrogéner le produit résultant.

7. Procédé de préparation d'un p-menth-6-ène-2-sulfonate, caractérisé en ce qu'il comprend l'étape consistant a faire réagir un alpha-pinène avec un sulfite ou bisulfite en présence d'un catalyseur de transfert de phase dans des conditions radicalaires, dans un milieu aqueux ou aqueux avec un cosolvant

organique à une température d'au moins 40°C jusqu'à la formation dudit p-menth-6-ène-2-sulfonate.

8. Procédé selon l'une quelconque des revendications 3 à 7, dans lequel le co-solvant organique est l'isopropanol ou l'éthanol.

9. Procédé selon l'une quelconque des revendications 3 à 8, dans lequel le rapport de l'eau au cosolvant organique est compris entre 1:100 et 100:1.

10. Procédé selon l'une quelconque des revendications 3 à 9, dans lequel on catalyse la réaction par des radicaux libres engendrés par un peroxyde organique ou minéral, un composé azoïque ou l'oxygéne.

11. Procédé selon la revendication 7, dans lequel le catalyseur de transfert de phase est un sel d'ammonium ou phosphonium quaternaire contenant au moins un groupe hydrophobe organique.

12. Procédé selon la revendication 7, dans lequel le peroxyde est le peroxybenzoate de t-butyle.